# EUROPEAN PATENT SPECIFICATION

(11) **EP 0 779 065 B1**
(45) Date of publication and mention of the grant of the patent: **19.03.2003**
(21) Application number: 96120117.5
(22) Date of filing: 13.12.1996
(51) Int. Cl.: A61F 13/15

(54) **Sanitary article capable to improve fit against the body and method and apparatus for manufacturing same**
Saugfähige Artikel mit verbessertem Sitz, Verfahren und Vorrichtung zu deren Herstellung
Articles absorbantes à capacité le rétention améliorée, méthode et appareil de fabrication

(30) Priority: 15.12.1995 US 573311
(43) Date of publication of application: 18.06.1997
(73) Proprietor: JOHNSON & JOHNSON INC., Montreal, Quebec H1N 2G4 (CA)
(72) Inventor: Brisebois, Henri, Lachenair, Quebec J6W 5W6 (CA); Costa, Rogerio, 12600-000 Lorena SP (BR); Hsieh, Tong-Ho, Marlboro, NJ 07746 (US); Salerno, Catherine E., Millington, NJ 07946 (US); Ulman, John, Woodbrindge, NJ 07095 (US)
(74) Representative: Groening, Hans Wilhelm, Dipl.-Ing.

(56) References cited:
- EP-A- 0 359 391
- DE-A- 3 512 859
- DE-U- 8 514 032
- GB-A- 1 487 682
- US-A- 4 643 727

## Description

### FIELD OF THE INVENTION

The present invention relates to novel disposable absorbent structures, in line with the preamble of claim 1. which are useful for absorbing body exudate. More particularly, the invention relates to disposable absorbent articles, such as sanitary napkins, diapers, panty liners, bandages, wound dressings, etc., which provide enhanced fit against the anatomy of the wearer in order to capture liquid discharges more effectively and thus reduce the incidence of failures. The present invention also relates to novel sanitary absorbent articles having a barrier layer construction which, in addition to its normal function of preventing liquids entrapped in the absorbent core of the sanitary article from escaping from a garment facing side thereof, is also capable of urging a body-facing surface of the sanitary article against the body of the user in response to pressure.

### BACKGROUND OF THE INVENTION

The ability of a sanitary absorbent article to efficiently capture and retain a discharge of body exudate is the result of a cooperative relationship between a plurality of superposed layers of materials having different liquid-absorbency characteristics. In its simplest form, a laminated absorbent article comprises a body facing liquid acquisition layer which may comprise an absorbent core, whose function is to acquire and permanently trap a liquid discharge and a barrier layer which prevents the trapped liquid from escaping from the absorbent core, and which may optionally be overlaid by a liquid permeable cover layer that in use contacts the skin of the wearer. Ideally, the cover layer should manifest a rapid and complete liquid take-up in order to capture, on contact, a liquid discharge. This feature is desirable for two reasons. Firstly, a rapid and complete liquid take-up reduces a potential risk of failure due to liquid leaking past the edges of the sanitary article or along the body in areas where there is no contact and staining the wearer's clothes. Secondly, the unpleasant sensation of wetness, due to prolonged contact between free body exudate in the process of being acquired by the cover layer and the skin, is diminished.

One factor which greatly influences the ability of an absorbent article to efficiently capture the liquid discharge is the quality of the interface between the body-facing surface and the body of the wearer. Indeed, if the body-facing surface is not in contact with the body, the liquid discharge has a tendency to pool on the surface of the sanitary article or travel along the body surface which increases the risk of failure and also contributes to discomfort. Moreover, normal body movements of the wearer, such as walking, have a tendency to compress and distort the sanitary article which further reduces the contact between a cover layer and the wearer's body.

Another factor involves the direction of fluid flow as it exits the body. The direction of fluid flow will vary greatly depending upon the position of the wearer, i.e., whether the wearer is upright, sitting, or fully reclined. For example, absorbent articles commonly fail during nighttime use where the wearer is in a fully reclined position for extended periods of time.

EP-A-0 359 391 discloses a moisture absorbent pad with a moisture impermeable thermoplastic substrate including gas bubbles with interstices between the bubbles for moisture absorbent material. A moisture impermeable inner film is adhered to portions of the substrate where the gas bubbles are formed. The gas bubbles act as a structural support to prevent release of substantial amounts of absorbed liquids from the absorbent material.

US 4,643,727 discloses an absorbent pad in line with the preamble of claim 1, said known pad having an intermediate plastic bubble layer covered on either side with a continuous layer of absorbent material wherein the bubble layer serves as a reservoir for a liquid that is siphoned around the edges of the bubble layer for storage. The air bubbles are uniformly spread about the bubble layer and projecting upwardly to present a barrier pattern to the incoming liquid and to break a liquid stream into a multitude of small streams which migrate to the edges of the bubbles and discharge into the lower layer for an even discharge across the surface of the absorbent layer.

DE-A-35 12 859 discloses an absorbent pad comprising an air cushion layer forming with an additional plastic layer container for the reception of fluid discharged by a wearer of the absorbent pad. The air cushions are designed to enlarge the volume for reception of fluid.

DE-A-85 14 032 discloses an absorbent pad having elastically stabilising air cushions. The air cushions may be uniformly distributed over the absorbent pad or may be in the form of longer channels. These channels may be welded longitudinally to compress the fluid contained in said channels. Moreover, perforations are provided with the cushions or channels to allow for pressure release.

There is therefor, a need for a sanitary napkin which provides continuous dynamic conformability to the body of the wearer throughout the entire time that the product is in use.

### SUMMARY OF THE INVENTION

It is an object of the present invention to provide a absorbent article which is capable of continuously dynamically conforming to the anatomy of the wearer.

It is another object of this invention to provide an absorbent article which reduces the incidence of failures due to liquid overflow and leakage.

It is another object of this invention to provide an absorbent structure which resists crushing and deformation due to lateral or other pressures applied by the thighs of the wearer.

It is another object of the present invention to provide an absorbent structure having a fluidly adaptive component which enhances contact between the absorbent structure and the body of the wearer, and thus provides the absorbent structure with the capability of more effectively absorbing body fluid exudate within the absorbent structure, thereby reducing the incidence of failures due to liquid overflow and leakage.

In accordance with the present invention, there has now been provided an absorbent structure having a body-facing liquid-acquisition layer and a fluid impervious, fluidly adaptive component capable of dynamically conforming the liquid-acquisition layer, at least locally, to the anatomy of the wearer by fulfilling the features of the characterizing part of claim 1.

### DETAILED DESCRIPTION OF THE INVENTION

The present invention is directed to an absorbent structure having a body-facing liquid-acquisition layer which possesses a dynamically variable surface configuration, and a fluid impervious, fluidly adaptive component, which upon the application of pressure thereto, is capable of dynamically conforming the liquid-acquisition layer, at least locally, to the anatomy of the wearer. The fluidly adaptive component is responsive to pressure applied to the component, wherein pressure exerted on a surface of one portion of the fluidly adaptive component causes some or all of the fluid in that portion to flow to a second adjacent portion of the fluidly adaptive component which experiences relatively less pressure than the first portion, whereby the volume of fluid in the second portion increases thus urging the surface of the second adjacent portion of the component to expand or bulge and thus urges the liquid-acquisition layer, which is in dynamicly responsive contact with the fluidly adaptive component, towards the wearer and to thus conform to the anatomy of the wearer. As used herein, the terminology "dynamically responsive contact" refers to either direct contact between the liquid acquisition layer and the fluidly adaptive component, as well as to indirect contact, where one or more intervening layers of material may be between the liquid acquisition layer and the fluidly adaptive component. In the case of indirect contact, it is important that the intervening layer(s) of material are capable of transmitting the pressure exerted by the expanding or bulging second portion of the fluidly adaptive component to the liquid acquisition layer. This may be effected in various ways including, but not limited to, using flexible materials, by providing hinge areas in flexible or non-flexible materials, by limiting the size of non-flexible materials such that pressure is exerted over a defined surface area which is generally less than the surface area of the liquid acquisition layer, and the like. Other suitable means of effecting the indirect transference of pressure between the fluidly adaptive component and the liquid acquisition layer are readily apparent to those skilled in the art and are also considered to be within the scope of this invention as defined by the appended claims.

The exact location of the fluidly adaptive component within the absorbent structure is not, per se, critical to the invention, provided of course, that it is located in an area of the absorbent structure which results in a tendency of the body-facing side of the absorbent structure to dynamically conform to the body of the wearer upon the application of pressure to at least one portion of the fluidly adaptive component. Advantageously, the fluidly adaptive component is located in or adjacent to a central area of the absorbent structure where body exudate is most likely to contact the absorbent structure. It has been found that when the fluidly adaptive component is located in a central portion of an absorbent structure such as a sanitary napkin, that lateral pressure applied to the sanitary napkin by a wearer's thighs, results in a preferential dynamic conformation of the body-contacting surface to the perineal area of the wearer's body.

In a preferred embodiment, the fluidly adaptive component is located directly adjacent to an area of the liquid acquisition layer which is intended to contact a discharge orifice of a wearer of the absorbent article, such that when the absorbent article is placed on the body of the wearer, body exudate emanating from the discharge orifice will preferentially contact the area of the liquid acquisition layer capable of being urged by the fluidly adaptive component into a body-contacting position.

The size of the fluidly adaptive component may vary widely, depending on the nature of the absorbent structure itself (sanitary napkin, wound dressing, etc.), the location of the fluidly adaptive component and whether there is a single fluidly adaptive component or a plurality of fluidly adaptive components. When there is a single fluidly adaptive component, it is preferably located in a central region of the absorbent structure, and is preferably substantially the same size as the central area of the absorbent structure. Alternatively, the fluidly adaptive component may extend either longitudinally, laterally, or both having a length and or width which is substantially equal to the length and/or width of the absorbent structure.

When the absorbent structure contains a plurality of individual fluidly adaptive components their individual sizes may be the same or different, and can advantageously be configured to dynamically conform large portions of the absorbent structure to the wearer's body, or alternatively may more precisely dynamically conform strategic areas of the absorbent structure to problem areas of a wearer's body. For example, side leakage is a common problem with conventional sanitary napkins. By providing one or more longitudinally oriented fluidly adaptive components along and adjacent to the longitudinal sides of the absorbent structure, a gasketing effect is obtained which prevents or inhibits side leakage. When a plurality of fluidly adaptive components are incorporated into an absorbent structure, they may be fluidly connected or may be isolated and completely separate so as to provide independent dynamic conformability to a number of areas of the absorbent structure. When fluid communication exists between a plurality of fluidly adaptive components, it is at least between two fluidly adaptive components that are at a finite distance from one another but which are in fluid communication through a passageway or opening connecting the fluidly adaptive components.

The fluidly adaptive components may have a number of shapes, sealing patterns and divisions. The divisions may take any number of forms such as simple sealed zones, complex multiple sealed zones with walls between the individual fluidly adaptive components. A fluidly adaptive component may adjacent to one or more fluidly adaptive components, i.e., fluid filled components may be adjacent to other fluid filled components, or they may be separated by non-fluid filled zones or other components of the absorbent article.

In accordance with a particularly preferred embodiment of the invention, an absorbent structure contains one fluidly adaptive component which is located in a center portion of the absorbent structure, and one or more fluidly adaptive components located in peripheral portions of the absorbent structure, i.e along the peripheral edge margins of the absorbent structure. As used herein, the terminology 'peripheral edge margins' refers not only to the placement of fluidly adaptive components into areas which are inward from the longitudinal sides of the absorbent structure, but also refers to placement of the fluidly adaptive components in appended structures such as wings, tabs, side cuffs, and the like, which are generally located outward from the longitudinal edges of a central absorbent core.

The fluid in the fluidly adaptive component may be one or a combination of the following fluidly adapting media: gases such as air, nitrogen and carbon dioxide, among others; liquids such as water and oils, among others, gels that are not too firm and that can flow in the fluidly adaptive component, and combinations of one or more of these media. The fluidly adaptive component may also contain, in addition to the fluid, some solid or semisolid substances or thixotropic gels. However, the nature and the amount of such substances should not be such as to prevent the fluid filled component from dynamically and transiently adapting to the contours of the user's body and clothing while the absorbent structure is being worn. It may be advantageous to use relatively high molecular weight fluids which less easily diffuse through polymeric film materials.

It is an important feature of the present invention that the fluidly adaptive component, upon the application of pressure thereto, preferentially urges the body facing surfaces of the absorbent structure towards the body of the user. This is generally effected by using a volume of fluid which is less than the total theoretical maximum volume for a given surface area of flexible material, i.e., the fluidly adaptive component is generally considered to be 'flaccid' in contrast to a pressurized fluid-containing component which is not capable of transferring fluid from a first zone of relatively high pressure to a second zone of relatively low pressure within the component. However, it is readily apparent that if an elastic material is used to form the fluidly adaptive component, it is possible to increase the volume of fluid within the fluidly adaptive component since this structure will elastically expand to provide the requisite transfer of fluid from a first zone to a second zone within the component.

In accordance with this aspect of the invention, the volume of fluid in the fluidly adaptive component is such as to allow for the fluid in one portion of the fluidly adaptive component to be transferable from that portion, area or zone of relatively high pressure to one or more portions, areas or zones of relatively lower pressure whereby the volume of fluid contained in these portions, areas or zones is increased, and thus are caused to expand or bulge. Fluid is thereby permitted to transfer from zones of high pressure and contact to zones of relatively low pressure and contact due to confining nature of a unified laminated absorbent structure, to the confined space between the user's body and clothing as well as to environmental causes due to contact with objects such as when a user sits or reclines. When fluid communication exists, it has the effect of moving fluid from a zone of relatively high pressure to a zone or zones of relatively low pressure in such quantity as to cause the zones of reduced contact to expand or bulge. This expansion causes the body-facing surface of the absorbent structure to more firmly engage the wearer's body, thereby effecting conformance of the absorbent structure to the wearer's anatomy. In this way, the fluidly adaptive component and the absorbent structure will continuously conform to the body of the wearer throughout the time that the absorbent structure is in use.

As discussed above, the volume of fluid within the fluidly adaptive component is less than the maximum potential volume for the component, and is preferably low enough for dynamic conformance of the absorbent structure to the body to occur, i.e., the fluidly adaptive component should be somewhat flaccid, rather than extremely firm, rigid and resistant to compression. The preferred volumes to achieve dynamic conformability may vary somewhat with the shape and volume of the fluidly adaptive component, the nature of the fluid (gas, liquid, gel, etc.), and the extensibility or elasticity of the material used to form the fluidly adaptive component. Without limiting the invention in any way, it has generally been found that an appropriate volume of fluid in a fluidly adaptive component formed from nonelastic film materials is typically from about 20% to 85%, preferably from about 35% to about 80% and most preferably about 75% of the theoretical maximum volume of the fluidly adaptive component. An appropriate volume is present in the fluidly adaptive component when superposed first and second layers, which define therebetween a cavity, is filled with a volume of fluid in an amount sufficient to maintain the layers in a separated or spaced apart configuration and when a portion of the first layer of the fluidly adaptive component, when subjected to pressure (such as e.g. by a wearer of a sanitary napkin during normal use), is capable of contacting the opposite second layer of the fluidly adaptive component.

It is considered an important feature of the present fluidly adaptive component that it possess the capability of confining the fluid throughout the expected lifetime of the product. This concept is described herein by the expression "fluid impervious fluidly adaptive component". It is of course understood that the exact meaning of this terminology may vary somewhat depending on the nature of the fluid present in the fluidly adaptive component. For instance, when the fluid is a gas, "fluid impervious" would be understood to mean the ability to prevent the gas from escaping the fluidly adaptive component, i.e. "gas impervious". On the other hand when the fluid is a liquid, "fluid impervious" shall mean "liquid impervious". In the case of gels, "fluid impervious" should mean the ability to prevent the gel from escaping the fluidly adaptive component. The fluidly adaptive component is impervious to the liquids intended to be absorbed by the absorbent structure, i.e. the fluidly adaptive component should preferably function in the presence of absorbed liquids (blood, urine, menstrual fluid, etc.) in the absorbent structure. As a result, the dynamically adaptive behavior of the absorbent structure continues to be maintained after repeated body fluid discharges. In accordance with this aspect of the invention, the fluidly adaptive component containing the fluid preferably comprises material which is substantially insoluble in body exudate.

The fluidly adaptive component is formed from two sheets of flexible fluid-impervious film, and more specifically, the fluidly adaptive component may be made of any or a combination of: extensible, elastic or stretchable materials, including latex rubber, silicone rubbers, polyurethanes, elastomeric block co-polymers such as styrene block copolymers. A preferred film derived from styrene block co-polymers is commercially available from the Shell Oil Company under the tradename KRATON. Other suitable flexible fluid-impervious films for use in forming the fluidly adaptive components of this invention include nonextensible, nonelastic, nonstretchable materials, including polyethylenes, elastomeric polyesters, nylons, co-extruded films such as polyethylene to ethylene vinyl acetate copolymers, as well as laminates of two or more layers of the foregoing flexible film materials.

The fluidly adaptive component may optionally comprise a separate article formed of materials additional to those of the absorbent structure. This embodiment is particularly advantageous when the absorbent structure is a disposable sanitary protection device for use in the perineal area of a user. In accordance with this aspect of the invention, the fluidly adaptive component comprises a separate insert which may be adhered onto a crotch portion of a wearer's undergarment, and then superposed with a conventional sanitary napkin, panty liner, adult incontinence device, and the like, onto a body facing surface of the fluidly adaptive component. The use of adhesives on a garment facing surface of the absorbent structure, optionally in combination with laterally extending wings or tabs may advantageously be incorporated onto the absorbent structure to maintain the absorbent structure within the crotch portion of a wearer's undergarment and to further shield the side portions of the wearer's undergarment.

Alternatively, the separately formed insert may be incorporated within the absorbent structure at some position, e.g., between a cover and a transfer layer, between an absorbent core and a barrier layer, between component layers of an absorbent core, etc. The insert may also be connectively adhered to at least one substructure of the absorbent structure at some position, e.g., to the barrier layer, the absorbent core or the transfer layer. Preferably, the fluidly adaptive component is integrally formed with the absorbent structure. In this embodiment of the invention, it is preferred that the fluidly adaptive component forms part of the barrier layer.

An important feature of the fluid filled component is its resiliency and ability to resist crushing and deformation. This provides the absorbent structure with enhanced stability and enhanced fit against the body of the wearer which translates into a reduced risk of failure. A particularly useful configuration which has been found to be effective in reducing sideways compression and crushing, is to locate the fluidly adaptive component in a configuration which extends transversely across a substantial portion of the absorbent structure. When incorporated into a sanitary napkin, this configuration helps resist side compression from the user's thighs during wear. In accordance with this embodiment, the absorbent structure is preferably a sanitary napkin and the fluidly adaptive component comprises one or more relatively narrow transverse channels or tubular pockets which extend transversely from one lateral edge of the sanitary napkin to the opposite lateral edge. The width of these relatively narrow channels is not, per se, critical to the invention,provided of course that they are sufficiently wide to effectively resist sideways compression of the absorbent structure. Typically, the width of these channels range from about 3 millimeters to about 5 centimeters These channels are resilient and have been found to inhibit lateral compression of the sanitary napkin and thus provide stability and deformation resistance against lateral compression to the sanitary napkin.

In another preferred embodiment of the present invention, the absorbent article is a disposable sanitary napkin comprising a body facing fluid permeable cover layer, a garment facing fluid impermeable barrier layer, and an absorbent core between the cover layer and the barrier layer. The sanitary napkin further comprises a fluidly adaptive component preferably located between the barrier layer and the absorbent core. In this embodiment the sanitary napkin has a fluidly adaptive component which is a generally elongate shape which is located in a central region of the absorbent structure, the central region occupying the approximate middle one third of the napkin's longitudinal dimensions. The fluidly adaptive component has a width which is equal to or slightly less that the width of the absorbent core, and a length which is sufficient to conform to the perineal area of a wearer of the sanitary napkin. The fluid impermeable barrier layer and the fluidly adaptive component may optionally comprise a unitary structure wherein two layers of polymeric material are laminated together in a superposed condition. The sheets are sealed to one another around their periphery, such as, e.g. by heat-sealing, to form a closed fluidly adaptive component filled with a volume of fluid, such as, e.g. air or liquid. When pressure is applied on a first portion of the fluidly adaptive component, some or all of the fluid therein is transferred to an adjacent second portion thereof which experiences less pressure than the first part, thus forming a bulge in the second portion. Since the sanitary napkin is in a relatively confined space defined by the perineal area of the wearer and the wearer's undergarments, the resultant bulge in the second portion of the fluidly adaptive component will have a tendency to dynamically conform the liquid-permeable cover toward the body of the wearer.

As embodied and broadly described herein, the invention also provides a sanitary napkin, comprising:
- a liquid-permeable cover layer for contact with the body of a wearer;
- an absorbent core located underneath the liquid-permeable cover layer and in liquid-communicative relationship therewith, the absorbent core being capable of absorbing a discharge of body exudate delivered on the liquid-permeable cover layer; and
- a liquid impermeable barrier for preventing liquid in the absorbent core from egressing the sanitary napkin from a garment facing surface thereof, the liquid-impermeable barrier including:
   a) first and second superposed layers defining therebetween a fluid-impervious fluidly adaptive component; and
   b) a fluid in the fluid-impervious fluidly adaptive component, whereby pressure exerted by the body of a wearer on a part of the fluidly adaptive component displaces the fluid to form a bulge tending to press the liquid-permeable cover layer towards the body of the wearer, the fluidly adaptive component being located underneath a vaginal orifice of a wearer when the sanitary napkin is placed on the body of the wearer, whereby body exudate discharged from the vaginal orifice has a tendency of contacting an area of the liquid-permeable cover layer capable of being urged upwardly by the fluidly adaptive component.

The surface of the absorbent structure that is intended to be worn against the body of the user (i.e the body facing surface) is covered by a layer of a body-fluid pervious material, typically referred to as a "cover layer". The cover layer may be formed from any fluid pervious material that is comfortable against the skin and that permits fluid to penetrate to the underlying core, which retains the fluid. The cover layer is preferably fluid repellent, i.e. it should retain little or no fluid in its structure to provide a relatively dry surface next to the skin. The fluid pervious cover layer may be a fibrous woven or nonwoven fabric made of fibers or filaments of polymers such as cotton, rayon, nylon, polyethylene, polypropylene, polyester, bi-component fibers of polyester sheathed in polyethylene or polypropylene, bi-component fibers of polypropylene sheathed in polyethylene, fibers of high melting polyester sheathed in low melting polyester, and combinations thereof.

In a most preferred embodiment, the cover layer may be formed from an apertured polymeric film. The thickness of the cover layer will vary from approximately 0.0254 to 1,57 mm (0.001 to 0.062 inch), preferably from 0,127 to 0,508 mm (0.005 to 0.020 inch) and most preferably from 0,254 to 0,381 mm (0.010 to 0.015 inch). Generally, the fluid pervious cover layer is a single sheet of material having a surface area sufficient to cover the body-facing surface of the absorbent article. Preferably, the fluid pervious cover layer is longer than the core so as to form the transverse ends. The transverse ends may be sealed with other pervious or non-pervious layers to fully enclose the central absorbent core.

The absorbent structure may further comprise a layer of a body fluid impervious material, typically referred to as a "barrier layer", on its garment facing surface. The fluid impervious barrier layer may comprise any thin, flexible, body fluid impermeable material and is typically a polymeric film such as, for example, polyethylene, polypropylene, cellophane, and the like. Alternatively, the barrier layer may be a normally fluid pervious material that has been treated to be impervious, such as impregnated fluid repellent paper, woven or nonwoven fabric material, closed cell flexible foams, such as polyurethane or cross-linked polyethylene. The fluid impervious barrier layer may optionally be permeable to gas including water vapor to provide an air breathable fluid barrier. The thickness of the barrier layer is not per se critical to the invention, provided of course that it is flexible. A typical barrier layer thickness, when formed from a polymeric film, is in the range of from 0.0005 to 0.025 inch and is preferably in the range 0.001 to 0.002 inch.

The barrier layer generally comprises a single sheet of material having a surface area sufficient to cover the entire garment-facing surface of the absorbent article. The fluid impervious barrier layer may extend around the sides of the core in a C-shaped configuration with the portions of the barrier layer that are adjacent its longitudinal edges extending upwardly from the garment facing surface toward the body facing surface so as to form a portion of the lateral sides of the absorbent structure.

The central portion of the absorbent structure contains an absorbent core. As is known in the art, the absorbent core may be comprised of a loosely associated absorbent hydrophilic material such as cellulose fibers, including wood pulp, wet or dry cross-linked wood pulp, curly wood pulp fibers, rayon fibers or cotton fibers, or other absorbent materials generally known in the art, including acrylate fibers, polyvinyl alcohol fibers, peat moss or super-absorbent particles or fibers. The absorbent core may also comprise synthetic fibers such as nylon, polyethylene, polypropylene, polyester, bi-component fibers of polyester sheathed in polyethylene or polypropylene, bi-component fibers of polypropylene sheathed in polyethylene, fibers of high melt polyester sheathed in low melt polyester, and combinations thereof. These fibers may be layered to form an absorbent core having a porosity gradient, and/or may be treated with a surfactant to make them hydrophilic and thereby wettable.

In a preferred embodiment, the central portion of the absorbent core has an hour glass shape and is thicker (i.e. in the "z" direction) in its center region than at its end portions, thus creating an absorbent article having a raised center portion. The relative thickness of the center region is not, per se, critical to the invention, and is generally from 1.1 to 4 times thicker than the transverse end regions of the absorbent core, preferably from 1.5 to 3 times thicker, and most preferably from 1.75 to 2.75 times thicker. The raised center portion has been found to provide enhanced body contact with the perineal area of the user's body, and thus enhances the transference of fluid from the user's body into the central absorbent core. In a most preferred embodiment, the raised center portion of the absorbent core further comprises an absorbent structure derived from sphagnum moss. Absorbent structures derived from sphagnum moss are more fully disclosed in U.S. Patent numbers 4,170,515 to Lalancette et al., 4,215,692, 4,226,237, and 4,507,122 to Levesque, 4,305,393 to Nguyen, 4,473,440 to Ovens, 4,618,496 to Brasseur, 4,676,871 to Cadieux et al., 4,992,324 to Dube, and 5,053,029 to Yang.

The absorbent structure of the present invention may optionally include a transfer layer between the fluid pervious cover layer and the absorbent core. The transfer layer generally comprises similar materials as those used in the absorbent core, but will have a more open porous structure than the central absorbent core having interstitial spaces between the fibrous materials which are greater than those in the absorbent core. This creates a porosity gradient which greatly enhances the rate of fluid transfer form the cover layer to the absorbent core which acts. as a reservoir for the absorbed fluids.

The absorbent structure is comprised of a longitudinally extending central portion having longitudinal sides and transverse ends. The central portion may have an approximately rectangular shape, an approximately oval shape, or preferably an hour-glass or dog-bone shape wherein the transverse ends are wider than the central portion of the absorbent article.

In accordance with yet another embodiment of the invention, the fluidly adaptive component may advantageously be used in combination with a deformation element of the type described more fully in European Patent Applications 0 335 252 and 0 335 253 to Buell. These references describe a disposable absorbent article having a flexure-resistant, deformation element. The deformation element has a convex upward configuration when the napkin is worn and pressed inward by the thighs. Upon the application of lateral compressive forces by the wearer's thighs, the deformation element forms a convex upward configuration. The combination of the fluidly adaptive component of the present invention with the above described deformation element provide a sanitary napkin with a controlled deformation upon the application of pressure to the lateral edges of the napkin. In this embodiment, the fluidly adaptive component may be located adjacent to a garment facing barrier layer, and is superposed on its body facing surface with the deformation element. The application of pressure to the fluidly adaptive component would not only urge the liquid acquisition layer towards the body of the wearer, but also maintain a convex upward configuration of the napkin.

Alternatively, the deformation element may be located adjacent to a garment facing barrier layer and the fluidly adaptive component may be superposed on its body-facing surface such that the application of lateral compressive forces to the deformation element would urge the fluidly adaptive component towards the body of the wearer and provide a dynamic conformance of the liquid acquisition layer.

As embodied and broadly described herein, the invention also provides a barrier layer for a disposable absorbent product, the barrier layer including a closed fluidly adaptive component containing a fluid, whereby pressure applied to a portion of the fluidly adaptive component causes fluid in that portion to flow to another portion of the component, resulting in an expansion or bulge in that portion.

As embodied and broadly described herein, the invention also provides a method for manufacturing a barrier layer for a sanitary disposable absorbent article, the method comprising the steps of:
- placing first and second fluid-impervious layers in a substantially superposed condition:

- subjecting at least one of the first and second fluid-impervious layers to a pressure differential for locally parting the layers to form a space therebetween;
- providing a fluid in the space,
- uniting the layers to one another around a periphery of the space in order to form a sealed fluidly adaptive component.

In a most preferred embodiment, the first and second fluid-impervious layers are placed between a pair of heat-sealing dies. The upper die has a surface configuration containing one or more three-dimensional fluidly adaptive component forming recesses. Each fluidly adaptive component forming recess is in fluid communication with a source of vacuum. When the source of vacuum is activated, a pressure differential is established in the fluidly adaptive component forming recesses which causes the first fluid-impervious layer (the one adjacent to the die with the fluidly adaptive component forming recesses) to conform to its three-dimensional surface configuration. The layers may be maintained in a separated orientation by means of injected fluid, tensioning, application of vacuum, and combinations thereof. As a result, the layers are locally parted in the areas of the fluidly adaptive component forming recesses.

A fluid is then introduced into the cavities defined by the fluidly adaptive component forming recesses. The dies are then closed to heat-seal the superposed layers around each fluidly adaptive component forming recess. Alternative sealing means include, but are not limited to adhesives, ultrasound, radio frequency and combinations thereof. As a result of this operation, the volume of fluid residing between the layers in the area of each fluidly adaptive component is completely sealed and can no longer escape.

As embodied and broadly described herein the invention also provides an apparatus for use in manufacturing a barrier layer of a disposable absorbent product, the apparatus comprising:
- a first die member including a fluidly adaptive component forming recess;
- at least one aperture in the fluidly adaptive component forming recess, the aperture being in fluid-communication with a source of vacuum, whereby when a first and second layers of fluid-impervious material in a substantially superposed condition are placed against the first die member and the source of vacuum is activated, a pressure differential is established in the fluidly adaptive component forming recess which causes a portion of one of the layers to be pulled in the fluidly adaptive component forming recess so as to locally part the layers and thus form a space therebetween; and
- a second die member, the first and second die members being capable of pressing the first and second layers for heat-sealing the first and second layers around the fluidly adaptive component forming recess in order to form a sealed fluidly adaptive component between the first and second layers.

While the inventive concept described herein is well-suited for sanitary napkins, it can also be applied to other absorbent articles such as diapers, adult disposable briefs, incontinence products and the like.

### BRIEF DESCRIPTION OF THE DRAWINGS

- Figure 1 is a perspective view of the sanitary napkin in accordance with the present invention, the napkin being shown in the inverted position so that the barrier layer is on top;
- Figure 2 is a top plan view of the sanitary napkin shown in Figure 1;
- Figure 3 is a cross-sectional view taken along lines 3-3 in Figure 1;
- Figure 4 is a schematic view in cross-section of a sanitary napkin placed against a body of the wearer depicting the shape adaptation manifested in response to downward pressure;
- Figure 5a is a vertical cross-sectional view of an apparatus for manufacturing the barrier layer of the sanitary napkin in accordance with the invention, two polymeric sheets being also shown prior to being processed in the apparatus;
- Figure 5b shows the apparatus of Figure 5a after completion of vacuum-molding process; and
- Figure 5c shows the apparatus of Figure 5b closed to heat-seal the two polymeric sheets to form closed fluidly adaptive components.

### DESCRIPTION OF A PREFERRED EMBODIMENT

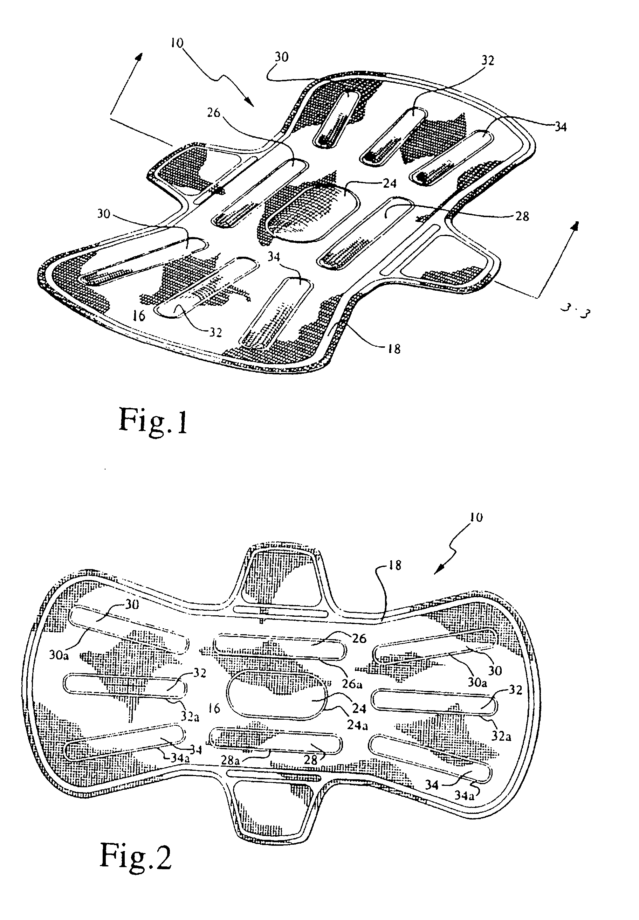

Referring to the annexed drawings, more particularly to Figures 1, 2 and 3, the present invention provides a sanitary napkin which is designated comprehensively by the reference numeral 10 and which is characterized by the ability to better conform to the surface of the wearer body. This feature enables the sanitary napkin to more efficiently capture a fluid discharge so as to limit the incidence of failures.

More specifically, the sanitary napkin 10 comprises a liquid-permeable cover layer 12 overlaying an absorbent core 14. The absorbent core 14 preferably comprises a pulp fluff material and may optionally include other absorbent materials or non-absorbent materials which aid in stabilizing the absorbent structure such as conjugate fibers, fusible fibers, binders, sphagnum peat moss particles, superabsorbents, and the like and combinations thereof. Underlying the absorbent core 14 is a barrier layer 16 comprising liquid-impervious film material so as to prevent liquid that is entrapped in the absorbent core from egressing the sanitary napkin and staining the wearer's undergarment. Note that in Figures 1 to 3, the sanitary napkin 10 is shown in an inverted position; its normal position of use being such that the cover layer 12 is on top and the barrier layer 16 on the bottom.

The cover layer 12 and the barrier layer 16 are joined along their marginal portions so as to form an enclosure that contains the absorbent layer 14. The joint may be made by means of adhesives, heat-bonding, ultrasonic bonding, radiofrequency sealing, mechanical crimping, and the like and combinations thereof. The peripheral seal line is shown in Figures 1 and 2 by the reference numeral 18.

The barrier layer 16 is characterized by the presence of a plurality of sealed air fluidly adaptive components which when compressed bulge and thus have a tendency to locally raise the absorbent core 14 and the cover layer 12 toward the body of the wearer so as to achieve a more intimate contact therewith. More specifically, the barrier layer 16 is a dual-layer construction comprising an upper layer 20 of a flexible polymeric material, such as polyethylene superposed over a lower sheet 22. For convenience, the upper layer and lower sheet are illustrated as two separate sheets. However, the fluidly adaptive component may optionally be made from a single sheet of film material which is folded into a 'C' configuration which is then sealed along the distal edge margins of the film material to form a continuous tube. Additional transverse seals would then define one or more fluidly adaptive components which may be discrete or in fluid communication with each other, depending upon whether the transverse seals completely bridged the distance between the folded edge margin and the distal edge seals. The sheets define therebetween a plurality of sealed flaccid fluidly adaptive components filled with a fluid, preferably air. More specifically, the barrier layer 16 includes a main oval shaped central fluidly adaptive component 24 flanked by a pair of narrow elongate side fluidly adaptive components 26 and 28. Additional fluidly adaptive components in the region of the longitudinal extremities of the sanitary napkin 10 are also provided. More particularly, three pairs of fluidly adaptive components 30, 32 and 34 originating near the central portion of the napkin 10 extend longitudinally towards the end portions of the sanitary napkin.

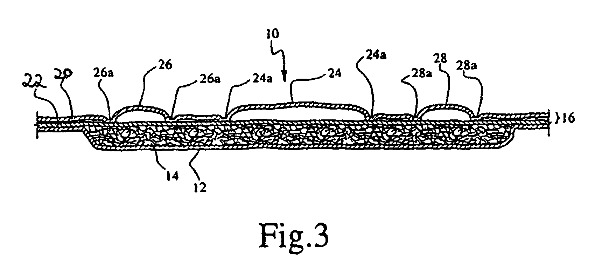

To form each fluidly adaptive component, the sheets 20 and 22 are sealed along one or more continuous and endless joint lines so that the fluid contained in the fluidly adaptive component is not capable of escaping. In the drawing, the joint line of each fluidly adaptive component is identified by the reference numeral used to designate the fluidly adaptive component followed by the suffix "a" (Figures 2 and 3).

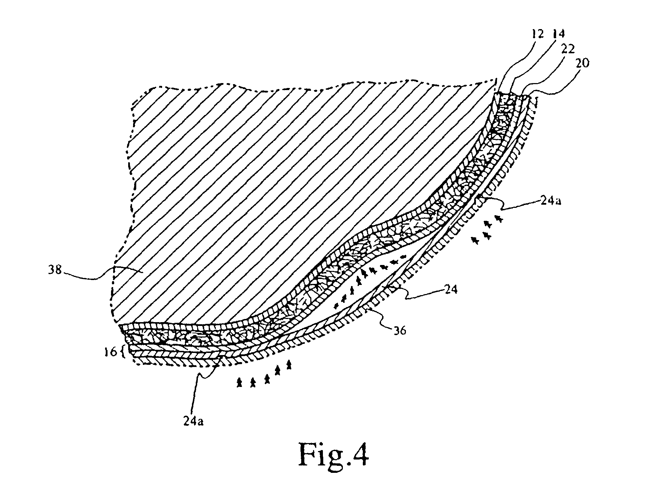

Figure 4 is a diagrammatic depiction of the perineal region of a wearer to which is applied the sanitary napkin 10, shown in cross-section. It should be noted that the illustration is very schematic and illustrates the sanitary napkin 10 with only the central fluidly adaptive component 24. The undergarment that maintains the sanitary napkin 10 against the perineal region of the wearer is shown by the reference numeral 36.

It will be appreciated that when a protruding portion 38 of the feminine anatomy presses on a first portion of the fluidly adaptive component 24, the fluid inside is displaced thus inflating a second portion of the fluidly adaptive component which experiences less pressure, and thus urges the absorbent core 14 and most importantly the cover layer 12 adjacent the second portion of the fluidly adaptive component into contact with the wearer's body. As a result, the sanitary napkins of the present invention provide a dynamic fit to the wearer's body resulting in a more intimate fluid-communicative relationship between the sanitary napkin 10 and the body of the user which reduces the incidence of failures.

The function of the remaining fluidly adaptive components 26, 28, 30, 32 and 34 is similar with the exception that these fluidly adaptive components act on the peripheral regions of the sanitary napkin rather than its center. The peripheral fluidly adaptive components also provide a gasketing effect by urging the edge portion of the napkin against the body of the wearer and thus inhibit or reduce the incidence of side leakage.

In an alternative embodiment, the sanitary napkin 10 can be provided with a single large fluidly adaptive component which is co-extensive with the absorbent core of the napkin. This embodiment is easy to manufacture and requires a relatively simply die to seal the polymeric sheets of the barrier layer 16. However, the use of multiple fluidly adaptive components in an arrangement where each fluidly adaptive component has a clearly delimited zone of influence, so pressure applied to a given fluidly adaptive component produces a reaction in that region only and not elsewhere has been found to provide a more selective adaptive characteristic to the absorbent structure. In contrast, in the single large fluidly adaptive component arrangement, pressure applied at some point on the absorbent structure will cause the entire surface of the absorbent structure surrounding the pressure application site to expand or bulge. The overall thickness of a single large fluidly adaptive component can be controlled by selectively adhering spaced apart portions of the upper surface of the fluidly adaptive component to the lower surface of the fluidly adaptive component in a manner similar to a quilted pattern. These seal patterns are preferably located at the distal ends of the fluidly adaptive component to provide a tapered end region.

In yet another embodiment of this invention, one or more central fluidly adaptive components 24 extend laterally to form one or more transversely extending bladders that provide crush resistance properties to the sanitary napkin. The napkin is capable of better resisting side compression and crushing from the user's thighs during wear.

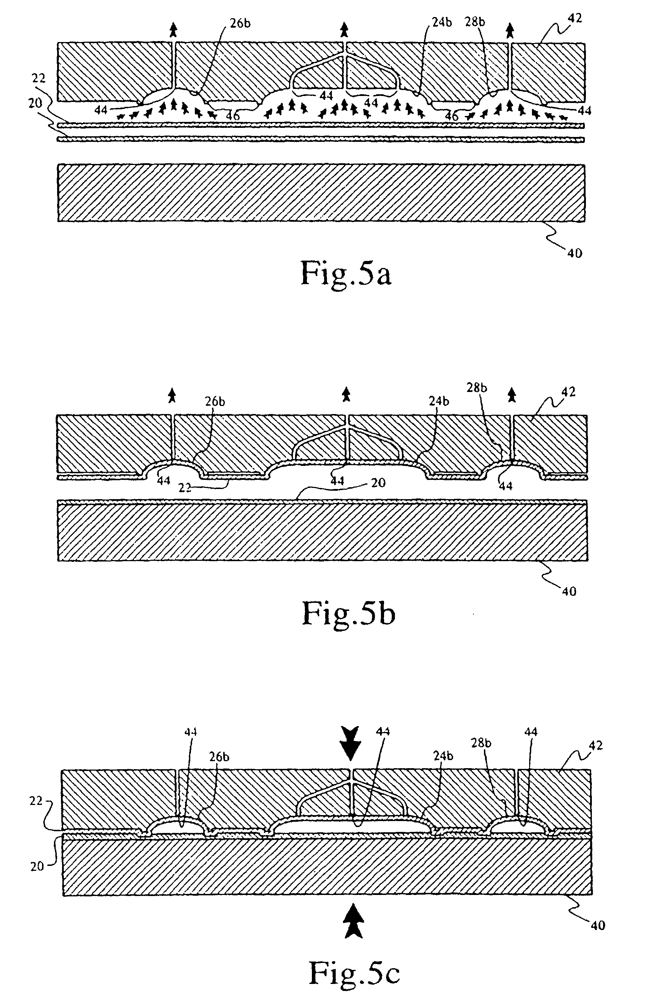

The method and the apparatus for manufacturing the barrier layer 16 will now be described in connection with Figures 5a to 5c. The apparatus comprises two dies 40 and 42 for sealing the superposed layers 20 and 22 in order to form the multi fluidly adaptive component arrangement. More specifically, the upper die 40 comprises a plurality of fluidly adaptive component forming recesses namely 24b, 26b and 28b to form the fluidly adaptive components 24, 26 and 28, respectively. Each fluidly adaptive component forming recess is provided with a plurality of orifices 44 that are connected to a vacuum pump (not shown) through channels in the die body.

To manufacture the barrier layer 16, the dies 40 and 42 are opened as shown in Figure 5a and the sheets of polyethylene 20 and 22 are inserted between them. The vacuum pump is then actuated to produce a pressure differential in the fluidly adaptive component forming recesses 24b, 26b and 28b. As a result, the sheet 22 is vacuum molded to conform to the three-dimensional topography of the die 42, as shown in Figure 5b.

The die 42 is also provided with a plurality of projections 46 corresponding to the seal lines surrounding the fluidly adaptive components. Both dies 40, 42 are heated (preferably by an impulse sealing element or resistive heating elements embedded in the die bodies) so when they are brought against one another to compress the sheets 20 and 22, the sheets are locally fused around the fluidly adaptive component forming recesses to seal the fluidly adaptive components and permanently entrap the air therein.

Finally the dies 40 and 42 are opened, the formed barrier layer is removed and it is processed in accordance to known techniques to assemble it to the remaining components of the sanitary napkin 10 so as to form a unitized structure.

Applications of the product of the present invention for sanitary and other health-care uses can be accomplished by any sanitary protection, incontinence, medical and absorbent methods and techniques as are presently or prospectively known to those skilled in the art. Thus, it is intended that the present application covers the modifications and variations of this invention provided that they come within the scope of the appended claims.

## Claims

1. An absorbent structure (10) having a longitudinal and a transverse axis as well as comprising:
- a body facing liquid-acquisition layer comprising a liquid-permeable cover layer (12) for contact with the body of a wearer and an absorbent core (14) located underneath the liquid-permeable cover layer (12) and in liquid-communicative relationship therewith, the absorbent core (14) being capable of absorbing a discharge of body exudate delivered on the liquid-permeable cover layer (12); and
- a component (24, 26, 28, 30, 32, 34) which is located underneath the absorbent core (14), is defined by first and second superposed layers (20, 22) sealed together about their peripheral edge margins (18, 24a, 26a, 30a, 32a, 34a) thereby defining a theoretical maximum volume and contains a fluid between the first and second superposed layers (20, 22) **characterized in that**
the component (24, 26, 28, 30, 32, 34) is fluid-impermeable, fluidly adaptive as well as in dynamically responsive contact with the liquid-acquisition layer; the fluid contained between the first and second superposed layers (20, 22) occupies a volume which is being less than the theoretical maximum volume for a given surface area of flexible material such that when in use pressure is exerted on a first portion of the fluid-impermeable, fluidly adaptive component (24, 26, 28, 30, 32, 34) the fluid can be displaced to a second portion of the fluid impermeable, fluidly adaptive component (24, 26, 28, 30, 32, 34) to expand the second portion and thereby urging and bulging at least a portion of the liquid-acquisition layer which is adjacent to the second portion of the fluid-impermeable, fluidly adaptive component (24, 26, 28, 30, 32, 34) as well as towards the body of a wearer of the absorbent structure (10).

2. The absorbent structure according to claim 1 wherein
the fluid-impermeable, fluidly adaptive component (24, 26, 28, 30, 32, 34) is on a garment facing side of the absorbent structure (10).

3. The absorbent structure according to claim 1 or 2 wherein
the fluid-impermeable, fluidly adaptive component (24) is located in a central portion of the absorbent structure (10), where body exudate is most likely to contact the absorbent structure (10).

4. The absorbent structure according to any of the preceding claims wherein
the fluid-impermeable, fluidly adaptive component (24) is located adjacent to an area of the liquid-acquisition layer which is intended to contact a discharge orifice of a wearer of the absorbent structure (10).

5. The absorbent structure according to any of the preceding claims wherein
the absorbent structure is a sanitary napkin or a unitary sanitary absorbent article (10), the fluid-impermeable, fluidly adaptive component (24, 26, 28, 30, 32, 34) thereof resulting in a dynamically variable surface configuration and further comprising a liquid-impermeable barrier (16) for preventing liquid in the absorbent core (14) from egressing the sanitary napkin (10) from a garment facing surface thereof.

6. The absorbent structure according to any of the preceding claims wherein
the absorbent structure (10) comprises a longitudinally extending central portion having longitudinally extending sides and transverse ends, and the fluid-impermeable, fluidly adaptive component (24, 26, 28, 30, 32, 34) extends longitudinally along and adjacent to at least one of the longitudinal sides or transversely from one longitudinal side to an opposite longitudinal side.

7. The absorbent structure according to any of the preceding claims wherein
the first and second layers (20, 22) define therebetween a plurality of fluid-impermeable, fluidly adaptive components (24, 26, 28, 30, 32, 34) located in a spaced apart relationship.

8. The absorbent structure according to any of the preceding claims,
including one centrally located fluid-impermeable, fluidly adaptive component (24) and at least one fluid-impermeable, fluidly adaptive component (26, 28, 30, 32, 34) located near a periphery (18) of the absorbent structure (10).

9. The absorbent structure according to any of the preceding claims wherein
the fluid-impermeable, fluidly adaptive component (24, 26, 28, 30, 32, 34) is capable of maintaining the liquid-acquisition layer in an elevated condition when in use the absorbent structure (10) is saturated with body exudate.

10. The absorbent structure according to any of the preceding claims wherein
the first and second superposed layers (20, 22) are bonded to one another along a continuous and endless seal line (18, 24a, 26a, 28a, 30a, 32a, 34a).

11. The absorbent structure according to any of the claims 5 to 10 wherein
the barrier layer (16) is underneath the absorbent core (14) and comprises the fluid-impermeable, fluidly adaptive component (24, 26, 28, 30, 32, 34).

## Patentansprüche

1. Eine absorbierende Struktur (10), welche eine longitudinale und eine transversale Achse hat, und außerdem umfassend:
- eine dem Körper zugewandte flüssigkeitsaumehmende Schicht, umfassend eine flüssigkeitsdurchlässige Deckschicht (12) für den Kontakt mit dem Körper eines Trägers und einen absorbierenden Kern (14), lokalisiert unterhalb der flüssigkeitsdurchlässigen Deckschicht (12) und mit dieser in flüssigkeitskommunizierender Beziehung, wobei der absorbierende Kern (14) in der Lage ist, einen auf die flüssigkeitsdurchlässige Deckschicht (12) gebrachten Ausfluß von Körperabsonderungen zu absorbieren; und
- eine Komponente (24, 26, 28, 30, 32, 34), die unterhalb des absorbierenden Kerns (14) lokalisiert ist, die definiert ist über erste und zweite aufeinander liegende Schichten (20, 22), die um ihre peripheren Kantenbereiche (18, 24a, 26a, 30a, 32a, 34a) miteinander versiegelt sind, dadurch ein theoretisches Maximalvolumen definierend, und die ein Fluid zwischen den ersten und zweiten aufeinander liegenden Schichten (20, 22) enthält, **dadurch gekennzeichnet, daß**
die Komponente (24, 26, 28, 30, 32, 34) Fluid-undurchlässig, Fluid-adaptiv und auch in dynamisch reagierendem Kontakt mit der flüssigkeitsaumehmenden Schicht ist; daß das zwischen den ersten und zweiten aufeinander liegenden Schichten (20, 22) enthaltene Fluid ein Volumen in Anspruch nimmt, das geringer ist als das theoretische Maximalvolumen für ein gegebenes Oberflächenareal eines flexiblen Materials, so daß, wenn beim Gebrauch Druck auf einen ersten Abschnitt der Fluid-undurchlässigen, Fluid-adaptiven Komponente (24, 26, 28, 30, 32, 34) ausgeübt wird, das Fluid zu einem zweiten Abschnitt der Fluid-undurchlässigen, Fluid-adaptiven Komponente (24, 26, 28, 30, 32, 34) verdrängt werden kann, um den zweiten Abschnitt zu expandieren und dadurch zumindest einen Abschnitt der flüssigkeitsaufriehmenden Schicht drängend und aufbauchend, der benachbart zu dem zweiten Abschnitt der Fluid-undurchlässigen, Fluid-adaptiven Komponente (24, 26, 28, 30, 32, 34) ist, und ebenfalls gegen den Körper eines Trägers der absorbierenden Struktur (10).

2. Die absorbierende Struktur nach Anspruch 1, wobei die Fluid-undurchlässige, Fluid-adaptive Komponente (24, 26, 28, 30, 32, 34) auf der dem Kleidungstück zugewandten Seite der absorbierenden Struktur (10) ist.

3. Die absorbierende Struktur nach Anspruch 1 oder 2, wobei die Fluid-undurchlässige, Fluid-adaptive Komponente (24) in einem zentralen Abschnitt der absorbierenden Struktur (10) lokalisiert ist, wo Körperabsonderung am wahrscheinlichsten mit der absorbierenden Struktur (10) in Kontakt tritt.

4. Die absorbierende Struktur nach einem der vorangehenden Ansprüche, wobei die Fluid-undurchlässige, Fluid-adaptive Komponente (24) benachbart zu einem Areal der flüssigkeitsaufnehmenden Schicht lokalisiert ist, die vorgesehen ist, um mit einer Ausflußöffnung des Trägers der absorbierenden Struktur (10) in Kontakt zu treten.

5. Die absorbierende Struktur nach einem der vorangehenden Ansprüche, wobei die absorbierende Struktur eine Binde oder einen einheitlichen absorbierenden Hygieneartikel (10) darstellt, die Fluid-undurchlässige, Fluid-adaptive Komponente (24, 26, 28, 30, 32, 34) desselben ergebend eine dynamisch variable Oberflächenkonfiguration, und des weiteren eine flüssigkeitsundurchlässige Barriere (16) umfaßt, um zu verhindern, daß Flüssigkeit aus dem absorbierenden Kern (14) der Binde (10) auf der dem Kleidungsstück zugewandte Fläche derselben austritt.

6. Die absorbierende Struktur nach einem der vorangehenden Ansprüche, wobei die absorbierende Struktur (10) einen sich longitudinal erstreckenden zentralen Abschnitt mit sich longitudinal erstreckenden Seiten und transversalen Enden umfaßt, und wobei die Fluid-undurchlässige, Fluid-adaptive Komponente (24, 26, 28, 30, 32, 34) sich longitudinal entlang und benachbart zu zumindest einer der longitudinalen Seiten oder transversal von einer longitudinalen Seite zu einer gegenüberliegenden longitudinalen Seite erstreckt.

7. Die absorbierende Struktur nach einem der vorangehenden Ansprüche, wobei die ersten und zweiten Schichten (20, 22) eine Mehrzahl an Fluid-undurchlässigen, Fluid-adaptiven Komponenten (24, 26, 28, 30, 32, 34) zwischen denselben, lokalisiert in beabstandetem Verhältnis, definieren.

8. Die absorbierende Struktur nach einem der vorangehenden Ansprüche, enthaltend eine zentral lokalisierte Fluid-undurchlässige, Fluid-adaptive Komponente (24) und zumindest eine Fluid-undurchlässige, Fluid-adaptive Komponente (26, 28, 30, 32, 34), lokalisiert nahe der Peripherie (18) der absorbierenden Struktur (10).

9. Die absorbierende Struktur nach einem der vorangehenden Ansprüche, wobei die Fluid-undurchlässige, Fluid-adaptive Komponente (24, 26, 28, 30, 32, 34) in der Lage ist, die flüssigkeitsaufnehmende Schicht in einem erhöhten Zustand aufrechtzuerhalten, wenn die absorbierende Struktur (10) im Gebrauch mit Körperabsonderung gesättigt ist.

10. Die absorbierende Struktur nach einem der vorangehenden Ansprüche, wobei die ersten und zweiten aufeinander liegenden Schichten (20, 22) miteinander entlang einer kontinuierlichen und endlosen Dichtungslinie (18, 24a, 26a, 28a, 30a, 32a, 34a) verbunden sind.

11. Die absorbierende Struktur nach einem der Ansprüche 5 bis 10, wobei die Barriereschicht (16) unterhalb des absorbierenden Kerns (14) ist und die Fluid-undurchlässige, Fluid-adaptive Komponente (24, 26, 28, 30, 32, 34) umfaßt.

## Revendications

1. Structure absorbante (10) ayant un axe longitudinal et transversal et comprenant également :
- une couche d'acquisition de liquide faisant face au corps comprenant une couche de couverture perméable aux liquides (12) pour le contact avec le corps d'un porteur et un noyau absorbant (14) situé au-dessous de la couche de couverture perméable aux liquides (12) et en relation de transmission de liquide avec celui-ci, le noyau absorbant (14) étant capable d'absorber une décharge d'exsudat corporel libéré sur la couche de couverture perméable aux liquides (12) ; et
- un composant (24, 26, 28, 30, 32, 34) qui est situé au-dessous du noyau absorbant (14), est défini par une première et une seconde couches superposées (20, 22) scellées ensemble autour de leurs marges de bords périphériques (18, 24a, 26a, 30a, 32a, 34a) définissant par-là un volume maximum théorique et contient un fluide entre la première et la seconde couches superposées (20, 22) **caractérisé en ce que**
le composant (24, 26, 28, 30, 32, 34) est imperméable aux fluides, s'adapte de manière fluide et a une réponse dynamique au contact avec la couche d'acquisition de liquide ; le fluide contenu entre la première et la seconde couches superposées (20, 22) occupe un volume qui est inférieur au volume maximum théorique pour une aire de surface donnée de matériau flexible tel que lorsqu'en utilisation une pression est exercée sur une première portion du composant imperméable aux fluides, s'adaptant de manière fluide (24, 26, 28, 30, 32, 34), le fluide peut être déplacé dans une seconde portion du composant imperméable aux fluides, s'adaptant de manière fluide (24, 26, 28, 30, 32, 34), pour dilater la seconde portion et par ce moyen presser et bomber au moins une portion de la couche d'acquisition de liquide qui est adjacente à la seconde portion du composant imperméable aux fluides, s'adaptant de manière fluide (24, 26, 28, 30, 32, 34), ainsi qu'en direction du corps d'un porteur de la structure absorbante (10).

2. Structure absorbante selon la revendication 1 dans laquelle
le composant imperméable aux fluides, s'adaptant de manière fluide (24, 26, 28, 30, 32, 34), est sur un côté de la structure absorbante (10) faisant face aux vêtements.

3. Structure absorbante selon la revendication 1 ou 2 dans laquelle
le composant imperméable aux fluides, s'adaptant de manière fluide (24), est situé dans une portion centrale de la structure absorbante (10), où l'exsudat corporel a le plus tendance à entrer en contact avec la structure absorbante (10).

4. Structure absorbante selon l'une quelconque des revendications précédentes dans laquelle
le composant imperméable aux fluides, s'adaptant de manière fluide (24), est situé de façon adjacente à une zone de la couche d'acquisition de liquide qui est destinée à être en contact avec un orifice de décharge d'un porteur de la structure absorbante (10).

5. Structure absorbante selon l'une quelconque des revendications précédentes dans laquelle
la structure absorbante est une serviette hygiénique ou un article absorbant sanitaire unitaire (10), le composant imperméable aux fluides, s'adaptant de manière fluide (24, 26, 28, 30, 32, 34), de celle-ci ayant pour résultat une configuration de surface variable dynamiquement et comprenant en outre une barrière (16) imperméable aux liquides pour empêcher un liquide dans le coeur absorbant (14) de sortir de la serviette hygiénique (10) pour aller dans un vêtement faisant face à sa surface.

6. Structure absorbante selon l'une quelconque des revendications précédentes dans laquelle
la structure absorbante (10) comprend une portion centrale s'étendant longitudinalement ayant des côtés s'étendant longitudinalement et des extrémités transversales, et le composant imperméable aux fluides, s'adaptant de manière fluide (24, 26, 28, 30, 32, 34), s'étend longitudinalement d'une manière adjacente et le long d'au moins un des bords longitudinaux ou transversalement d'un bord longitudinal à un bord longitudinal opposé.

7. Structure absorbante selon l'une quelconque des revendications précédentes dans laquelle
la première et seconde couches (20, 22) définissent entre elles une multiplicité de composants imperméables aux fluides, s'adaptant de manière fluide (24, 26, 28, 30, 32, 34), situées dans une relation d'espacement relatif.

8. Structure absorbante selon l'une quelconque des revendications précédentes,
comprenant un composant imperméable aux fluides, s'adaptant de manière fluide (24), situé en position centrale et au moins un composant imperméable aux fluides, s'adaptant de manière fluide (26, 28, 30, 32, 34), situé près d'une périphérie (18) de la structure absorbante (10).

9. Structure absorbante selon l'une quelconque des revendications précédentes dans laquelle
le composant imperméable aux fluides, s'adaptant de manière fluide (24, 26, 28, 30, 32, 34), est capable de maintenir la couche d'acquisition de liquide dans un état élevé lorsqu'en utilisation la structure absorbante (10) est saturée avec l'exsudat corporel.

10. Structure absorbante selon l'une quelconque des revendications précédentes dans laquelle
la première et seconde couches superposées (20, 22) sont collées l'une à l'autre le long d'une ligne de scellement continue et sans fin (18, 24a, 26a, 28a, 30a, 32a, 34a).

11. Structure absorbante selon l'une quelconque des revendications 5 à 10 dans laquelle
la couche barrière (16) est au-dessous du noyau absorbant (14) et comprend le composant imperméable aux fluides, s'adaptant de manière fluide (24, 26, 28, 30, 32, 34).
